# EUROPEAN PATENT SPECIFICATION

(11) **EP 1 968 542 B1**
(45) Date of publication and mention of the grant of the patent: **14.08.2013**
(21) Application number: 06824193.4
(22) Date of filing: 18.12.2006
(51) Int. Cl.: A61K 9/10

(54) **SOLID DISPERSION COMPRISING AN ACTIVE INGREDIENT HAVING A LOW MELTING POINT AND TABLET FOR ORAL ADMINISTRATION COMPRISING SAME**
FESTE DISPERSION MIT EINEM WIRKSTOFF MIT NIEDRIGEM SCHMELZPUNKT UND DIESEN ENTHALTENDE TABLETTE ZUR ORALEN VERABREICHUNG
DISPERSION SOLIDE COMPRENANT UN INGREDIENT ACTIF A BAS POINT DE FUSION ET COMPRIME DESTINE A L'ADMINISTRATION ORALE COMPRENANT CETTE DISPERSION SOLIDE

(30) Priority: 16.12.2005 KR 20050124386
(43) Date of publication of application: 17.09.2008
(73) Proprietor: Hanmi Science Co., Ltd., Hwaseong-si, Gyeonggi-do (KR)
(72) Inventor: WOO, Jong Soo, Suwon-si, Gyeonggi-do 440-300 (KR); KIM, Sang Wook, Daejeon 305-340 (KR); YI, Hong Gi, Suwon-si, Gyeonggi-do 441-708 (KR); RYU, Jae Kuk, Suwon-si, Gyeonggi-do 441-360 (KR)
(74) Representative: Viering, Jentschura & Partner
(86) International application number: PCT/KR2006/005526
(87) International publication number: WO 2007/069874

(56) References cited:
- WO-A1-00/56339
- WO-A1-97/02017
- WO-A2-01/41733
- DE-A1- 19 509 807
- US-A- 4 806 359
- US-A- 4 806 359
- US-A- 4 867 987
- US-B1- 6 391 338
- US-B1- 6 787 157
- TAKEUCHI H ET AL: "Solid dispersion particles of amorphous indomethacin with fine porous silica particles by using spray-drying method" INTERNATIONAL JOURNAL OF PHARMACEUTICS, ELSEVIER BV, NL LNKD- DOI:10.1016/J.IJPHARM.2004.12.019, vol. 293, no. 1-2, 11 April 2005 (2005-04-11), pages 155-164, XP004791675 ISSN: 0378-5173
- "Colloidal Silicon Dioxide, Crospovidone, Lactose, Starch" In: Kibbe A.H.: "Handbook of Pharmaceutical Excipients" 31 December 2000 (2000-12-31), American Pharmaceutical Association , London , XP002577667 vol. 3, * the whole document *

## Description

### Field of the Invention

The present invention relates to tabletting process comprising a fused solid dispersion comprising an active ingredient having a low melting point, and a tablet for oral administration comprising same.

### Background of the Invention

Non steroidal anti-inflammatory drugs such as ibuprofen or dexibuprofen (S(+)-ibuprofen) having low melting points, tend to melt by the heat generated during a compress tabletting process, causing the problems of capping and sticking, particularly when the drug content is high. In order to prevent such problems, a relatively high amount of excipient needs to be used but, in this case, the dosage unit must be increased to achieve an effective plasma concentration of the active ingredient.

Accordingly, there have been reported numerous methods for effectively compressing such a low-melting active ingredient into a tablet. For example, WO 92/020334 and DE 3,922,441 disclose a pharmaceutical composition comprising an ibuprofen or dexibuprofen salt, and WO 93/004676 discloses a pharmaceutical composition comprising ibuprofen agglomerates using a suspension comprising ibuprofen or a salt thereof, starch, surfactant, water and a solvent. WO 01/41733 describes a melting process for the preparation USAID granules having advantageous tabletting properties.

WO 95/001781 discloses a method for preparing a bilayer tablet consisting of a rapid release layer and a controlled release layer, wherein the rapid release layer comprises ibuprofen, corn starch, cross-linked polyvinylpyrrolidone, carboxymethyl starch, magnesium stearate, while the controlled release layer comprises ibuprofen, mannitol, hydroxypropylmethyl cellulose, talc, magnesium stearate and colloidal silica.

However, the above methods are not to fully satisfactory in solving the problems of capping and sticking that occur during a compression tabletting process.

### Summary of the Invention

Accordingly, it is an object of the present invention to provide a process for preparing a fused solid dispersion comprising an active ingredient having a low melting point which can be easily compressed into a tablet.

It is another object of the present invention to provide a process for preparing a tablet for oral administration comprising same, which is capable of maintaining uniform release of the drugs over a long period of time.

In accordance with one aspect of the present invention, there is provided a process for preparing a fused solid dispersion comprising an active ingredient having a melting point of 80 °C or below and a pharmaceutically acceptable absorbent having a specific surface area ranging from 20 to 400 m²/g.

In accordance with one aspect of the present invention, there is provided a process for preparing a controlled release tablet for oral administration comprising the fused solid dispersion.

In accordance with another aspect of the present invention, there is provided a process for preparing a multilayer tablet for oral administration consisting of a rapid release layer and a controlled release layer containing the fused solid dispersion.

In accordance with still another aspect of the present invention; there is provided a process for preparing a tablet for oral administration comprising:
(a) heating to melt an active ingredient having a melting point of 80 °C or below and adding a pharmaceutically acceptable absorbent having a specific surface area ranging from 20 to 400 m²/g thereto to obtain a homogenous fused solid dispersion;
(b) cooling, drying and pulverizing the fused solid dispersion obtained in step (a) to obtain granules; and
(c) adding a release-controlling agent or a pharmaceutically acceptable excipient to the granules obtained in step (b) and compressing the resulting mixture into a tablet.

### Brief Description of Drawings

Fig. 1: *the in vitro* dissolution profiles of the rapid release tablets prepared in Examples 6 to 10 of the present invention;
Fig. 2: *the in vitro* dissolution profiles of the controlled release tablet prepared in Example 11 of the present invention as well as those of the bilayer tablets consisting of a rapid release layer and a controlled release layer prepared in Examples 12 and 13 of the present invention;
Fig. 3: the variation in the *in vitro* dissolution profile of the tablet prepared in Example 12 of the present invention as function of the rate of the paddle rotation;
Fig. 4: *the in vitro* dissolution profiles of the bilayer tablets consisting of a rapid release layer and a controlled release layer prepared in Examples 14 to 16 of the present invention; and
Fig. 5: the variation in *the in vitro* dissolution profile of the tablet prepared in Example 16 of the present invention as function of the rate of the paddle rotation.

### Detailed Description of the Invention

The tablet for oral administration comprises a controlled release tablet comprising a fused solid dispersion containing an active ingredient and a release-controlling agent, a rapid release tablet comprising the fused solid dispersion and a pharmaceutically acceptable excipient, and a multilayer tablet for oral administration having a controlled release layer formed using ingredients for the controlled release tablet and a rapid release layer, using ingredients for the rapid release tablet.

Each ingredient of the tablet is described in detail as follows:

### <Fused solid dispersion>

The fused solid dispersion of the present invention comprises an active ingredient having a melting point of 80 °C or below and one or more pharmaceutically acceptable absorbent having a specific surface area ranging from 20 to 400 m²/g. The fused solid dispersion may further comprise a tabletting aid selected from the group consisting of a sugar alcohol, a water soluble polymer, an oily base and a mixture thereof. The weight ratio of the active ingredient : the pharmaceutically acceptable absorbent : the tabletting aid preferably ranges from 1 : 0.01∼3 : 1∼2.

### (1) Active ingredient

In the present invention, the active ingredient used in the fused solid dispersion has a melting point of 80 °C or below, preferably 50 to 80 °C, and representative examples of the active ingredient include ibuprofen (melting point : 75 ∼ 77 °C), dexibuprofen (melting point : 50 ∼ 54°C) or a mixture thereof which are non steroidal anti-inflammatory drugs useful in the treatment of a rheumatoid arthritis

### (2) Pharmaceutically acceptable absorbent

In the present invention, the pharmaceutically acceptable absorbent used in the fused solid dispersion may be any of those conventionally used in the pharmaceutical field, and representative examples of the absorbent include light anhydrous silicic acid, hydrotalcite, aluminum magnesium silicate, aluminum hydroxide, aluminum silicate, magnesium aluminum methasilicate, bentonite, lactose, dextrin, starch, microcrystalline cellulose, hydroxypropylmethyl cellulose, hydroxypropyl cellulose, hydroxyethyl cellulose, ethyl cellulose, methyl cellulose, polyethylene glycol, finely-divided cross-linked polyvinylpyrrolidone or a mixture thereof.

Particularly, in order to avoid the problems such as capping and sticking occurring due to melting of the active ingredient by the heat generated during compression tabletting, a pharmaceutically acceptable absorbent having a specific surface area ranging from 20 to 400 m²/g, preferably, 100 to 300 m²/g, more preferably, 150 to 250 m²/g is used. When the range of the specific surface area of pharmaceutically acceptable absorbent is less than the lower limit, the capping and sticking problems still occur. In accordance with the present invention, the weight ratio of the active ingredient and the absorbent may range from 1:0.01 ∼ 3, preferably, from 1:0.1 ∼ 2. The absorbent may be added after heating to melt the active ingredient.

### (3) Tabletting aid (Sugar alcohol, water soluble polymer, oily base or mixture thereof)

In order to facilitate the granulation after grinding and increase the binding efficiency of the granules during compress tabletting by way of diminishing the melting fixation, the inventive fused solid dispersion may further comprise a tabletting aid selected from the group consisting of a sugar alcohol, a water soluble polymer, an oily base and a mixture thereof. The weight ratio of the active ingredient : the tabletting aid preferably ranges 1:0∼2.

Representative examples of the sugar alcohol used in the present invention include xylitol, sorbitol, mannitol and a mixture thereof, representative examples of the water soluble polymer include hydroxypropylmethyl cellulose, hydroxypropyl cellulose, polyvinylpyrrolidone, polyethylene glycol, polyethylene oxide, polyvinyl alcohol and a mixture thereof, and the representative examples of the oily base include sucrose fatty acid ester, glyceryl behenate, glyceryl palmitostearate, glyceryl monooleate, glyceryl monostearate and a mixture thereof.

The fused solid dispersion according to the present invention may be prepared using any conventional mixer, preferably a universal mixer or a heat-melt extruder.

The method of preparing the fused solid dispersion with a universal mixer or the heat-melt extruder is described in detail as follows:

### (a) Preparation of the fused solid dispersion using a universal mixer

The active ingredient is added to a universal mixer preheated to 60 °C to 100 °C and heat-melted, followed by mixing homogeneously. A pharmaceutically acceptable absorbent having a specific surface area ranging from 20 to 400 m²/g is added to the melten drug and the mixture is stirred for 20 to 60 minutes to obtain a homogeneous dispersion. At this time, a tabletting aid such as a sugar alcohol, a water soluble polymer and an oily base may be further added the dispersion. After shutting down the heater, the dispersion is stirred at room temperature, and the resulting agglomerate is collected and dried by cold blasting to obtain a fused solid dispersion comprising the active ingredient. The fused solid dispersion thus obtained is ground with a high-speed grinder and the resulting granules are filtered through No. 14 mesh (1410 *µ*m) to 20 mesh (850 µm), preferably 20 mesh (850 *µ*m) to obtain a fused solid dispersion.

### (b) Preparation of the fused solid dispersion with a heat-melt extruder

The active ingredient and the pharmaceutically acceptable absorbent having a specific surface area ranging from 20 to 400 m²/g are homogeneously mixed, and the mixture placed in a loading hopper is heated to melt in the hot compression screw chamber, followed of extruding the melt. The obtained agglomerate is homogeneously mixed with kneader-mixer, and the mixtures are filtered through a screen to obtain a fused solid dispersion having a uniform size.

In this process, the length of the time the active ingredient is exposed to the heat is shortened, and a fused solid dispersion having a uniform size distribution can be obtained. Thus, a fused solid dispersion having a uniform size distribution can be manufactured by a less time-consuming single process which is conducted by carrying out the inputting, melting and screening of the active ingredients in sequence.

### < Tablet comprising the fused solid dispersion>

Various types of tablets such as controlled release tablet, rapid release tablet and multilayer tablet can be prepared by optionally adding a pharmaceutically acceptable excipient to the fused solid dispersion and compressing into a tablet without the use of a cooler. The compressed tablet preferably has a hardness in the range from 4 to 16 kp, preferably 8 to 12 kp.

### (A) Controlled release tablet

A controlled release tablet comprises the above-mentioned fused solid dispersion and a release-controlling agent and may further comprises a pharmaceutically acceptable excipient. The weight ratio of the fused solid dispersion : the release-controlling agent : the pharmaceutically acceptable excipient ranges from 1 :0.01 ∼3:0∼3, and preferably, from 1:0.05∼2:0.01∼2.

### (A-1) Release-controlling agent

The release-controlling agent for maintaining uniform release rate for a long period of time can be selected from the group consisting of polyethylene oxide having a molecular weight ranging from 10,000 to 9,000,000, hydroxypropylmethyl cellulose having a molecular weight ranging from 1,000 to 4,000,000, hydroxypropyl cellulose, carboxyvinyl polymer, polyvinyl alcohol, xanthan gum, guar gum, locust bean gum, carboxymethyl cellulose and its derivative, methyl cellulose and its derivative, and povidone-polyvinylacetate copolymer having a molecular weight ranging from 2,000 to 2,000,000. In accordance with the present invention, the weight ratio of the fused solid dispersion : release-controlling agent may range from 1 : 0.01 ∼3, and preferably, from 1 : 0.05∼2.

### (A-2) Pharmaceutically acceptable excipient

In the present invention, in order to maintain an appropriate hardness and dosage form of a tablet, the controlled release tablet may further comprise a pharmaceutically acceptable excipient.

The pharmaceutically acceptable excipient used in the present invention may be used any conventional one used in the pharmaceutical field, and representative examples of the pharmaceutically acceptable excipient include a cross-linked polyvinylpyrrolidone, a cross-linked sodium carboxymethyl cellulose, carboxymethyl starch, calcium methacrylate-divinylbenzene copolymer, polyvinyl alcohol, lactose, microcrystalline cellulose and cellulose derivative, starch and its derivative, cyclodextrin and dextrin derivative, pregelatinized starch and its derivative, colloidal silica, magnesium stearate, glyceryl monostearate, sodium stearyl fumarate, talc, and hydrogenated caster oil.

In accordance with the present invention, the weight ratio of the fused solid dispersion : the pharmaceutically acceptable excipient may range from 1 : 0 ∼3, and preferably, from 1 : 0.01∼2.

### (B) Rapid release tablet

In the present invention, a rapid release tablet comprises the above-mentioned fused solid dispersion, and the above-mentioned pharmaceutically acceptable excipient used in the controlled release tablet. The weight ratio of the fused solid dispersion : the pharmaceutically acceptable excipient may range from 1 : 0.05 ∼3, and preferably, from 1 : 0.1∼2.

### (C) Multilayer tablet

A multilayer tablet in accordance with the present invention may be prepared by forming a controlled release layer with ingredients of the controlled release tablet and by forming a rapid release layer with ingredients of the rapid release tablet to manipulate the release of the active ingredient.

The bilayer tablet consisting of the rapid release tablet and the controlled release layer can be prepared by subjecting the ingredient for the rapid release layer to a first tablet compression step, depositing the ingredients for the controlled release layer thereon, and subjecting the resulting mixture to a second tablet compression step. The tablet compression process of the controlled release layer does not always have to be carried out after tabletting the rapid release layer. The tablet compression of the controlled release layer can be carried out first, and then the granules of the rapid release layer are added thereto, followed of tablet compression. Also the rapid release layer and the controlled release layer can be sequentially or reversely filled, which is compressed into a tablet in one step.

The multilayer tablet of the present invention can be also prepared as a trilayer tablet consisting of rapid release and controlled release layers.

When the tablet consisting of the rapid release layer and the controlled release layer comprising the same active ingredient according to the present invention is subjected to *in vitro* release tests in accordance with the paddle method at 100 rpm (Korea pharmacopoeia 8^{th} ed. *in vitro* dissolution tests 2^{nd} method) using 900mL of artificial gastric fluid(Korea pharmacopoeia 8^{th} ed., the 2 ^{nd} solution for the disintegrating-test), 85% or more of the active ingredient of the rapid release layer preferably is released within about 1 hour after initiating the test, while the active ingredient of the controlled release layer is released sequentially, preferably in amounts corresponding to 1 to 30% within about 1 hour, 30 to 70% within about 5 hours, and 85% or more within 12 hours after initiating the test.

The span of the release time of the active ingredient of the controlled release layer or the controlled release tablet can be prolonged by controlling the type and amount of excipient used in the controlled release layer. When *in vitro* dissolution tests were conducted according to the above method, the active ingredient of the rapid release layer in the multilayer tablet is released, preferably in amounts corresponding to 1 to 30% within 1 hour, 30 to 70% within 6 hours, 60 to 90% within 12 hours, 80% or more within 24 hours after initiating the test.

The rapidly-released active ingredient allows the plasma drug concentration to promptly reach the effective treating level while the slowly-released active ingredient can maintain the effective plasma drug concentration during the intended time. Thus, the pharmaceutically useful tablet according to the present invention is easily prepared without being hindered by such problems as capping and sticking during the course of compression tabletting, which can be effectively implemented in a large-scale manufacturing process.

The following Examples are intended to further illustrate the present invention without limiting its scope.

### Examples

### <Preparation of fused solid dispersion>

### Example 1

300 g of dexibuprofen was added to a universal mixer (VERSATILE MIXER (250DM-rrs), DALTON) preheated to 60 °C and was allowed to melt, followed by mixing homogeneously. 60 g of light anhydrous silicic acid having a specific surface area of 200±25 m²/g was slowly added thereto, and the mixture was stirred for 45 minutes to obtain a homogeneous dispersion (*see* Table 1). The resulting dispersion was cooled to the room temperature while stirring to obtain a solid dexibuprofen dispersion agglomeration. The resulting agglomeration was cooled to the room temperature by cold blasting (30 °C) for about 2 hours, and the resulting product was then ground with a high-speed grinder. The resulting granules were filtered through No. 20 mesh (850*µ*m) to obtain a fused solid dispersion.

### Example 2

A fused solid dispersion was prepared by repeating the procedure of Example 1 except for using 110 g of light anhydrous silicic acid having a specific surface area of 200±25 m²/g.

### Example 3

A fused solid dispersion was prepared by repeating the procedure of Example 1 except for using 110 g of light anhydrous silicic acid having a specific surface area of 300±25 m²/g.

### Example 4

A fused solid dispersion was prepared by repeating the procedure of Example 1 except that 300 g of dexibuprofen and 50 g of xylitol were added to the Universal mixer preheated to 95 °C and melted. 60 g of light anhydrous silicic acid having a specific surface area of 200±25 m²/g was slowly added thereto and the mixture was stirred for 45 minutes to obtain a homogeneous dispersion.

### Example 5

A fused solid dispersion was prepared by repeating the procedure of Example 4 except for using 20 g of hydroxypropylmethyl cellulose instead of 50 g ofxylitol.

**Table 1**

| Ingredients | Example1 (g) | Example 2 (g) | Example 3 (g) | Example 4 (g) | Example 5 (g) |
|---|---|---|---|---|---|
| Dexibuprofen | 300 | 300 | 300 | 300 | 300 |
| Light anhydrous Silicic acid (200±25m²/g) | 60 | 110 | - | 60 | 60 |
| Light anhydrous Silicic acid (200±25m²/g) | - | - | 110 | - | - |
| Xylitol | - | - | - | 50 | - |
| Hydroxypropylmethyl cellulose | - | - | - | - | 20 |
| Total | 360 | 410 | 410 | 410 | 380 |

### <Preparation of rapid release tablet>

### Example 6

In accordance with the components listed in Table 2, 205 mg of the fused solid dispersion obtained in Example 3 (amount of dexibuprofen : 150 mg per tablet), 10 mg of lactose, 49.7 mg of microcrystalline cellulose, 3.8 mg of cross-linked sodium carboxymethyl cellulose, and 5.1 mg of light anhydrous silicic acid as a pharmaceutically acceptable excipient were mixed together for 60 minutes and 11.4 mg of talc as a lubricant was added thereto. The resulting mixture was stirred for 5 minutes and compressed to a hardness of about 8 to 12 kp to obtain a rectangular rapid release tablet.

### Examples 7 to 10

Fused solid dispersions were prepared by repeating the procedure of Example 6 using the component listed in Table 2.

**Table 2**

| Ingredients | | Example 6 (mg) | Example 7 (mg) | Example 8 (mg) | Example 9 (mg) | Example 10 (mg) |
|---|---|---|---|---|---|---|
| Fused Solid dispersion (amount of dex X-ibuprofen) | Example 2 | - | 205.0 (150) | 136.7 (100) | - | - |
| | Example 3 | 205.0 (150) | - | - | - | - |
| | Example 4 | - | - | - | 683.3 (500) | - |
| | Example 5 | - | - | - | - | 380 (300) |
| Excipient | Lactose | 10 | - | 6.7 | 33.4 | - |
| | Ludipress^{®} (BASF) | - | - | - | - | 95.0 |
| | Microcrystalline cellulose | 49.7 | - | 33.1 | 165.8 | - |
| | Hydroxypropyl cellulose | - | 3.8 | - | - | - |
| | Micro Shellac^{®} 100 (MEGLE) | - | 132 | - | - | - |
| | Cross-linked sodium Carboxymethyl cellulose | 3.8 | 36.2 | 2.5 | 12.5 | 20 |
| | Light anhydrose Silicic acid | 5.1 | - | 3.4 | - | - |
| Lubricant | Magnesium stearate | - | 3.6 | - | 5.8 | 5 |
| | Talc | 11.4 | - | 7.6 | 24.2 | - |
| Total | | 285 | 380.6 | 190 | 925 | 500 |

### Test Example 1: In vitro dissolution test of rapid release tablet

The rapid release tablets prepared in Examples 6 to 10 were each subjected to an *in vitro* dissolution test based on Korea Food and Drug Administration (KFDA) and Release Guidelines on the drug for oral administration, and the release pattern was analyzed under the following conditions.

### <Dissolution test method>

Samples : Rapid release tablets prepared in Examples 6 to 10
Test solution: The disintegrating-test 2nd method described in Korea pharmacopoeia, pH 6.8 artificial gastric fluid, 900 mL, 37 ± 0.5 °C
Dissolution method: The dissolution test method described in Korea pharmacopoeia (the paddle method), rotation speed: 50 rpm

**Table 3**

| Dissolution time(min) | Dissolution rate (%) | | | | |
|---|---|---|---|---|---|
| | Example6 | Example7 | Example8 | Example9 | Example10 |
| 5 | 53.5 ± 4.0 | 62.8 ± 2.9 | 60.5 ± 1.5 | 59.0 ± 8.1 | 66.7 ± 2.6 |
| 10 | 69.9 ± 5.5 | 85.9 ± 1.3 | 80.6 ± 3.7 | 76.8 ± 1.7 | 89.1 ± 2.7 |
| 15 | 79.1 ± 4.8 | 93.7 ± 0.6 | 93.5 ± 2.1 | 91.6 ± 0.5 | 94.9 ± 1.1 |
| 30 | 94.7 ± 2.1 | 96.2 ± 0.6 | 98.5 ± 1.0 | 95.6 ± 4.9 | 95.6 ± 1.9 |
| 45 | 100.0 ± 0.3 | 98.9 ± 0.1 | 99.8 ± 1.0 | 100.8. ± 1.4 | 96.6 ± 2.2 |
| 60 | 101.1 ± 0.7 | 98.8 ± 0.1 | 100.5 ± 0.5 | 101.2 ± 2.4 | 97.6 ± 1.4 |

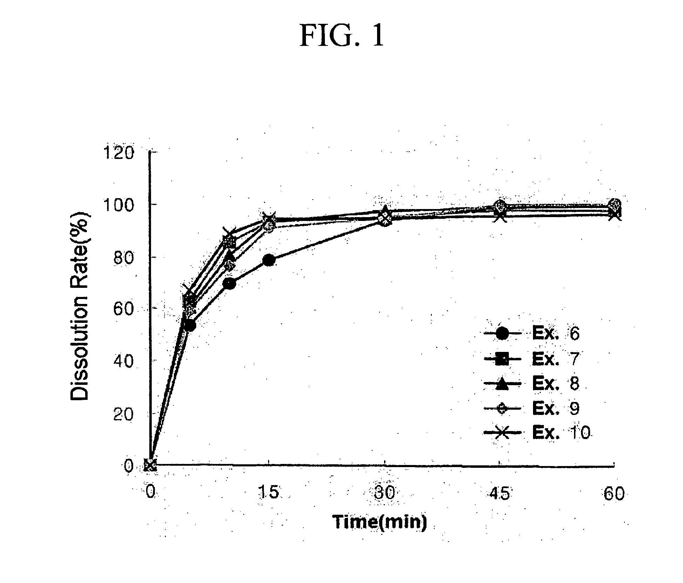

As can be seen from Table 3 and Fig. 1, each of the rapid release tablets prepared in Examples 6 to 10 showed a rapid drug release pattern (85% or more within 30 minutes after initiating release of the drug), and thus the inventive rapid release tablet comprising the inventive fused solid dispersion as an active ingredient provides rapid therapeutical effects.

### Example 11: Preparation of controlled release tablet

A controlled release tablet was prepared by repeating the procedure of Example 6 except that the fused solid dispersion, the release-controlling agent, and the lubricant listed in Table 4 were used.

**Table 4**

| Ingredients | | Example 11 |
|---|---|---|
| Fused solid dispersion (amount of dexibuprofen) | Example 5 | 231.8 (183.0 mg) |
| Release-controlling agent | Hydroxypropylmethyl cellulose 2208, 4000SR | 35.0 |
| | Calcium phosphate dibasic | 74.2 |
| | Xanthangum | 28.0 |
| | Locust bean gum | 7.0 |
| | Micro shellac^{®} 100 | 30.0 |
| | Light anhydrous silicic acid | 24.0 |
| Lubricant | Magnesium stearate | 4.8 |
| Total | | 434.8 |

### Test Example 2: In vitro dissolution test of controlled release tablet

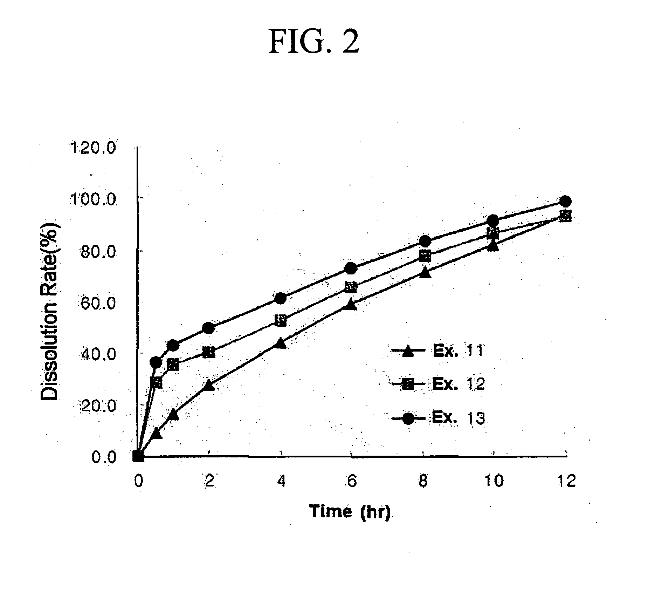

The controlled release tablet prepared in Example 11 was subjected to *in vitro* dissolution test under the following conditions, and the results are shown in Table 5 and Fig. 2.

### <Dissolution test method>

Sample : Controlled release tablet prepared in Example 11
Test solution: The disintegrating-test 2nd method described in Korea pharmacopoeia, pH 6.8 artificial gastric fluid, 900 mL, 37 ± 0.5 °C
Dissolution method: the dissolution test method described in Korea pharmacopoeia (the paddle method), rotation speed: 100 rpm

**Table 5**

| Dissolution time(hr) | Dissolution rate(%) |
|---|---|
| 0.5 | 9.2 ± 1.2 |
| 1 | 16.5 ± 1.1 |
| 2 | 28.0 ± 1.1 |
| 4 | 44.3 ± 0.7 |
| 6 | 59.6 ± 1.1 |
| 8 | 72.0 ± 0.8 |
| 10 | 82.6 ± 2.4 |
| 12 | 93.9 ± 2.6 |

As can be seen in Table 5 and Fig. 2, the controlled release tablet prepared in Example 11 slowly released the active ingredient of the controlled release portion over a period of 12 hours.

### Examples 12 and 13: Preparation of bilayer tablets consisting of rapid release and controlled release layers (1)

The components listed in Table 6 were mixed together and the mixture was subjected to a first tablet compression step to a hardness of about 2 to 3 kp, and then, the controlled release layer was deposited thereon and the resulting material was subjected to a second tablet compression step to a hardness of about 8 to 12 kp to obtain bilayer tablets.

**Table 6**

| Ingredients | | | Example 12 (mg) | Example 13 (mg) |
|---|---|---|---|---|
| Rapid-release layer | Resulting mixture of Example 7 (dexibuprofen: 150.0 mg) | | 365.6 | - |
| | Resulting mixture of Example 8 (dexibuprofen; 100.0 mg) | | - | 190.0 |
| Controlled-release layer | Fused solid dispersion (amount of dexibuprofen) | Example 2 | 478.3 (350.0 mg) | 239.2 (175.0 mg) |
| | Release-controlling agent | Polyethylene oxide (Molecular weight: 5.000.000) | 23.3 | 37.5 |
| | | Calcium phospate, dibasic | 76.1 | 36.2 |
| | | Hydroxy propyl cellulose | 9.0 | 5.5 |
| | Lubricant | Magnesium stearate | 9.0 | - |
| | | Talc | - | 13.3 |
| Total | | | 961.3 | 521.7 |

### Test Example 3: In vitro dissolution test of bilayer tablet (1)

*In vitro* dissolution tests were conducted using the bilayer tablets prepared in Examples 12 and 13 under the following condition, and the results are shown in Table 7 and Fig. 2.

### <Dissolution test method>

Samples : Bilayer tablets prepared in Examples 12 and 13
Test solution : The disintegrating-test 2nd method described in Korea pharmacopoeia, pH 6.8 artificial gastric fluid, 900 mL, 37 ± 0.5 °C
Dissolution method : The dissolution test method described in Korea pharmacopoeia (the paddle method), rotation speed: 100 rpm

**Table 7**

| Dissolution time(hr) | Dissolution rate(%) | |
|---|---|---|
| | Example 12 | Example 13 |
| 0.5 | 28.6 ± 1.5 | 36.5 ± 2.8 |
| 1 | 35.7 ± 1.1 | 43.1 ± 2.4 |
| 2 | 40.5 ± 1.0 | 50.1 ± 2.2 |
| 4 | 53.0 ± 0.2 | 61.7 ± 2.2 |
| 6 | 66.1 ± 1.7 | 73.4 ± 2.0 |
| 8 | 78.2 ± 2.6 | 83.7 ± 0.9 |
| 10 | 87.1 ± 2.8 | 91.8 ± 1.1 |
| 12 | 93.4 ± 2.4 | 99.1 ± 0.2 |

As can be seen in Table 7 and Fig. 2, each of the bilayer tablets prepared in Examples 12 and 13 showed that all the active ingredient of the rapid release portion was released, regardless of the amount of the active ingredient, and thereafter, the active ingredient of the controlled release portion was slowly released over a period of 12 hours.

### Test Example 4: In vitro dissolution test of bilayer tablet (1) as function of the rotation number

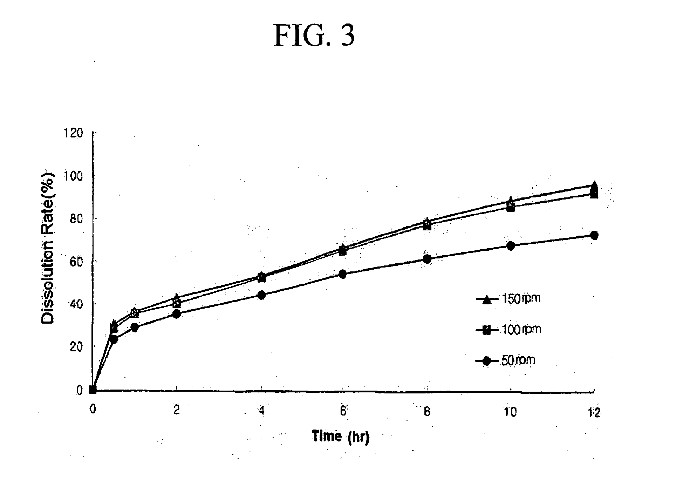

An *in vitro* dissolution test was conducted using the bilayer tablet prepared in Example 12 under the following conditions, and the results are shown in Table 8 and Fig. 3.

### <Dissolution test method>

Sample : Bilayer tablet prepared in Example 12
Test solution: The disintegrating-test 2nd method described in Korea pharmacopoeia, pH 6.8 artificial gastric fluid, 900 mL, 37 ± 0.5 °C
Dissolution method: The dissolution test method described in Korea pharmacopoeia (the paddle method), rotation speed: 50, 100 and 150 rpm

**Table 8**

| Dissolution time(hr) | Dissolution rate (%) | | |
|---|---|---|---|
| Revolution per minute(RPM) | 50 rpm | 100 rpm | 150 rpm |
| 0.5 | 23.4 ± 0.9 | 28.6 ± 1.5 | 30.6 ± 0.3 |
| 1 | 28.8 ± 0.9 | 35.7 ± 1.1 | 36.2 ± 0.9 |
| 2 | 35.6 ± 0.2 | 40.5 ± 1.0 | 43.1 ± 2.0 |
| 4 | 44.9 ± 1.0 | 53.0 ± 0.2 | 54.0 ± 0.4 |
| 6 | 54.9 ± 2.3 | 66.1 ± 1.7 | 67.4 ± 0.3 |
| 8 | 62.4 ± 2.6 | 78.2 ± 2.6 | 80.2 ± 0.3 |
| 10 | 69.0 ± 2.7 | 87.1 ± 2.8 | 89.6 ± 0.2 |
| 12 | 74.4 ± 3.4 | 93.4 ± 2.4 | 97.5 ± 0.3 |

As can be seen from Table 8 and Fig. 3, the tablet rapidly released the drug during the initial 1 hour to provide prompt therapeutical effects, regardless of the rotation speed, and thereafter the tablet displayed a steady release pattern of the drug, suitable for maintaining continuous therapeutical effects.

### Examples 14 to 16 : Preparation of bilayer tablet (2)

The components of the controlled release listed in Table 9 were subjected to a first tablet compression step to a hardness of about 2 to 3 kp, and then, the rapid release layer was deposited thereon, and the resulting material was subjected to a second tablet compression step to a hardness of about 8 to 12 kp to obtain bilayer tablets.

**Table 9**

| | | | Example 14 (mg) | Example 15 (mg) | Example 16 (mg) |
|---|---|---|---|---|---|
| Rapid release layer | Resulting mixture of Example 8 (dexibuprofen : 100.0 mg) | | 190.0 | 190.0 | 190.0 |
| Controlled-release layer | Fused solid dispersion (amount of dexibuprofen) | Example2 (175.0 mg) | 239.2 | 239.2 | 239.2 |
| | Release Controllng agent | Polyethylene oxide. (Molecular weight: 5.000.000) | 52.0 | 73.5 | 37.5 |
| | | Xanthangum | - | - | 11.0 |
| | | Locust bean gum | - | - | 3.5 |
| | | Calcium phospate, dibasic | 36.2 | 36.2 | 36.2 |
| | | Hydroxypropyl cellulose | 5.5 | 5.5 | 5.5 |
| | Lubricant | Talc | 13.3 | 13.3 | 13.3 |
| Total | | | 536.2 | 557.7 | 536.2 |

### Test Example 5: In vitro dissolution test of bilayer tablet (2)

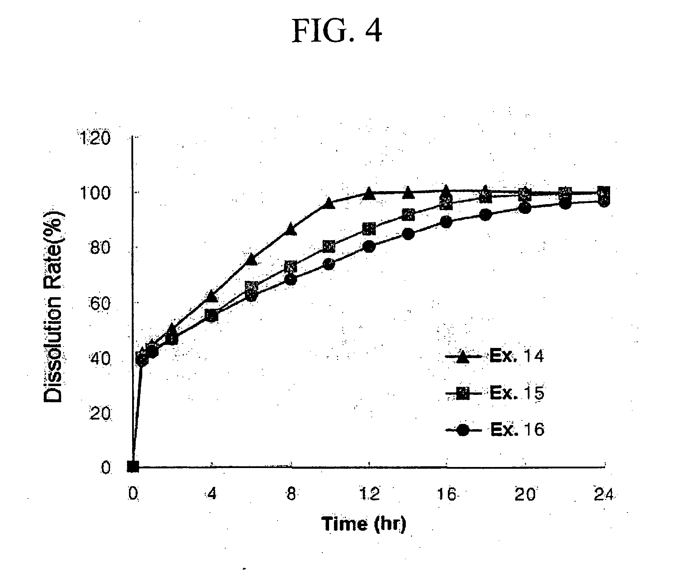

*In vitro* dissolution tests were conducted using the bilayer tablets prepared in Examples 14 to 16 under the following conditions, and the results are shown in Table 10 and Fig. 4.

### <Dissolution test method>

Samples : Bilayer tablets prepared in Examples 14 and 16
Test solution: The disintegrating-test 2nd method described in Korea pharmacopoeia, pH 6.8 artificial gastric fluid, 900 mL, 37 ± 0.5 °C
Dissolution method: The dissolution test method described in Korea pharmacopoeia (the paddle method), rotation speed: 100 rpm

**Table 10**

| Dissolution time(hr) | Dissolution rate (%) | | |
|---|---|---|---|
| | Example 14 | Example 15 | Example 16 |
| 0.5 | 41.5 ± 1.6 | 39.9 ± 0.4 | 39.0 ± 0.4 |
| 1 | 44.8 ± 1.8 | 42.7 ± 0.6 | 42.1 ± 0.6 |
| 2 | 50.4 ± 1.9 | 46.6 ± 0.1 | 46.6 ± 0.7 |
| 4 | 62.6 ± 3.2 | 55.3 ± 1.1 | 54.8 ± 0.6 |
| 6 | 75.7 ± 5.6 | 65.4 ± 2.8 | 62.5 ± 0.7 |
| 8 | 87.2 ± 7.1 | 73.1 ± 3.7 | 68.5 ± 0.5 |
| 10 | 96.5 ± 5.1 | 80.6 ± 4.0 | 74.0 ± 0.1 |
| 12 | 100.2 ± 2.6 | 87.0 ± 3.6 | 80.5 ± 1.8 |
| 14 | 100.7 ± 2.1 | 92.5 ± 2.8 | 85.3 ± 1.9 |
| 16 | 100.9 ± 2.2 | 96.3 ± 2.0 | 89.7 ± 1.2 |
| 18 | 100.7 ± 2.0 | 98.6 ± 1.3 | 92.4 ± 0.8 |
| 20 | 100.5 ± 1.0 | 99.5 ± 0.5 | 95.0 ± 0.5 |
| 22 | 100.5 ± 1.9 | 99.9 ± 1.1 | 96.8 ± 1.2 |
| 24 | 100.6 ± 1.5 | 100.5 ± 0.9 | 97.5 ± 0.9 |

As can be seen from Table 10 and Fig. 4, all the active ingredient of rapid release layer was released within 1 hour, regardless of the amount of the active ingredient, and thereafter, the tablet released the active ingredient continuously for 12 to 24 hours.

### Test Example 6: In vitro dissolution test of bilayer tablet (2) as function of the rotation number

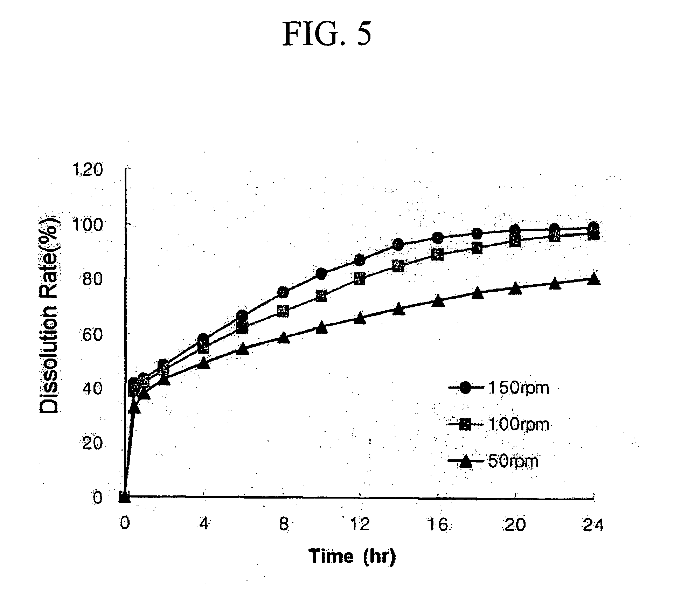

*In vitro* dissolution test was conducted using the bilayer tablet prepared in Example 16 under the following conditions, and the results are shown in Table 11 and Fig. 5.

### <Dissolution test method>

Sample : Bilayer tablet prepared in Example 16
Test solution: The disintegrating-test 2nd method described in Korea pharmacopoeia, pH 6.8 artificial gastric fluid, 900 mL, 37 ± 0.5 °C
Dissolution method: The dissolution test method described in Korea pharmacopoeia (the paddle method), rotation speed: 50, 100 and 150 rpm

**Table 11**

| Dissolution time(hr) | Dissolution rate(%) | | |
|---|---|---|---|
| Revolution per minute(RPM) | 50 rpm | 100 rpm | 150 rpm |
| 0.5 | 32.9 ± 0.3 | 39.0 ± 0.4 | 41.6 ± 0.2 |
| 1 | 38.1 ± 1.4 | 42.1 ± 0.6 | 43.5 ± 0.5 |
| 2 | 43.4 ± 2.2 | 46.6 ± 0.7 | 48.5 ± 0.4 |
| 4 | 49.5 ± 2.7 | 54.8 ± 0.6 | 57.9 ± 1.9 |
| 6 | 54.5 ± 2.6 | 62.5 ± 0.7 | 66.7 ± 2.7 |
| 8 | 58.8 ± 2.8 | 68.5 ± 0.5 | 75.3 ± 3.8 |
| 10 | 62.7 ± 2.8 | 74.0 ± 0.1 | 82.4 ± 3.9 |
| 12 | 66.1 ± 2.8 | 80.5 ± 1.8 | 87.7 ± 4.1 |
| 14 | 69.6 ± 2.9 | 85.3 ± 1.9 | 93.0 ± 3.2 |
| 16 | 72.8 ± 2.9 | 89.7 ± 1.2 | 95.7 ± 2.4 |
| 18 | 75.6 ± 2.7 | 92.4 ± 0.8 | 97.3 ± 2.1 |
| 20 | 77.6 ± 1.5 | 95.0 ± 0.5 | 98.9 ± 1.1 |
| 22 | 79.2 ± 2.1 | 96.8 ± 1.2 | 99.2 ± 1.5 |
| 24 | 81.1 ± 0.9 | 97.5 ± 0.9 | 99.8 ± 0.5 |

As can be seen from Table 11 and Fig. 5, the tablet rapidly released the drug in the initial 1 hour to provide fast therapeutical effects, followed by a steady release pattern of the drugs suitable for continuously maintaining the therapeutical effect.

Accordingly, as shown from the result of the dissolution test, the active ingredient of the rapid release portion was rapidly released within the initial 1 hour to attain an effective blood concentration thereof, exerting fast therapeutical effects. The active ingredient of the controlled release portion was slowly released over a period of 12 to 24 hours, thereby maintaining an effective concentration of the drug in the blood at a constant level during the intended time.

## Claims

1. A process for preparing a tablet for oral administration comprising :
(a) heating to melt an active ingredient being ibuprofen, dexibuprofen (S(+)-ibuprofen), or a mixture thereof and adding a pharmaceutically acceptable adsorbent being light anhydrous silicic acid having a specific surface area ranging from 20 to 400 m²/g thereto to obtain a homogenous fused solid dispersion;
(b) cooling, drying and pulverizing the fused solid dispersion obtained in step (a) to obtain granules; and
(c) adding a release-controlling agent or a pharmaceutically acceptable excipient to the granules obtained in step (b) and compressing the resulting mixture into a tablet.

2. The process of claim 1, which further comprises the step of adding a tabletting aid selected from the group consisting of a sugar alcohol, a water soluble polymer, an oily base and a mixture thereof, during the addition of light anhydrous silicic acid in step (a).

3. The process of claim 1, wherein the fused solid dispersion comprises the active ingredient and the pharmaceutically acceptable adsorbent in a weight ratio ranging from 1 : 0.01 to 1 : 3.

4. The process of claim 2, wherein the fused solid dispersion comprises the active ingredient and the tabletting aid in a weight ratio ranging 1 : 0 to 1 : 2.

5. The process of claim 2, wherein the sugar alcohol is selected from the group consisting of xylitol, sorbitol, mannitol and a mixture thereof.

6. The process of claim 2, wherein the water soluble polymer is selected from the group consisting of hydroxypropylmethyl cellulose, hydroxypropyl cellulose, polyvinylpyrrolidone, polyethylene glycol, polyethylene oxide, polyvinyl alcohol and a mixture thereof.

7. The process of claim 2, wherein the oily base is selected from the group consisting of sucrose fatty acid ester, glyceryl behenate, glyceryl palmitostearate, glyceryl monooleate, glyceryl monostearate and a mixture thereof.

8. The process of claim 1, wherein the tablet for oral administration is a controlled release tablet comprising the fused solid dispersion and a release-controlling agent for the slow release of the active ingredient.

9. The process of claim 8, wherein the tablet for oral administration further comprises a pharmaceutically acceptable excipient.

10. The process of claim 9, wherein the tablet for oral administration comprises the fused solid dispersion, the release-controlling agent and the pharmaceutically acceptable excipient in a weight ratio ranging from 1 : 0.01 ∼3 : 0-3.

11. The process of claim 1, wherein the tablet for oral administration is a multilayer tablet consisting of a rapid release layer comprising the fused solid dispersion and the pharmaceutically acceptable excipient, and a controlled release layer comprising the fused solid dispersion and a release-controlling agent.

12. The process of claims 8 or 11, wherein the release-controlling agent is selected from the group consisting of polyethylene oxide having a molecular weight ranging from 10,000 to 9,000,000, hydroxypropylmethyl cellulose having a molecular weight ranging from 1,000 to 4,000,000, hydroxypropyl cellulose, carboxyvinyl polymer, polyvinyl alcohol, xanthan gum, guar gum, locust bean gum, carboxymethyl cellulose and its derivative, methyl cellulose and its derivative, and povidone-polyvinylacetate copolymer having a molecular weight ranging from 2,000 to 2,000,000.

13. The process of claim 11, wherein the controlled release layer further comprises a pharmaceutically acceptable excipient.

14. The process of any one of claims 9, 11 and 13, wherein the pharmaceutically acceptable excipient is selected from the group consisting of a cross-linked polyvinylpyrrolidone, a cross-linked sodium carboxymethyl cellulose, carboxymethyl starch, calcium methacrylate-divinylbenzene copolymer, polyvinyl alcohol, lactose, microcrystalline cellulose and cellulose derivative, starch and its derivative, cyclodextrin and dextrin derivative, pregelatinized starch and its derivative, colloidal silica, magnesium stearate, glyceryl monostearate, sodium stearyl fumarate, and hydrogenated caster oil.

15. The process of claim 11, wherein the rapid release layer comprises the fused solid dispersion and the pharmaceutically acceptable excipient in a weight ratio ranging from 1 : 0.05 to 1 : 3.

16. The process of claim 13, wherein the controlled release layer comprises the fused solid dispersion, the release-controlling agent and the pharmaceutically acceptable excipient in a weight ratio ranging from 1 : 0.01 - 3 : 0 - 3.

## Patentansprüche

1. Ein Verfahren zur Bereitstellung einer Tablette zur oralen Verabreichung umfassend:
(a) Erhitzen bis zum Schmelzen eines aktiven Bestandteils, der Ibuprophen, Dexibuprofen (S(+)-Ibuprophen) oder ein Gemisch davon ist, und Hinzufüge eines pharmazeutisch annehmbaren Adsorptionsmittels, das leichte wasserfreie Kieselsäure mit einer spezifischen Oberfläche von 20 bis 400 m²/g ist, um eine homogen verschmolzene feste Dispersion zu erhalten;
(b) Kühlen, Trocknen und Pulverisieren der verschmolzenen festen Dispersion erhalten in Schritt (a), um Granula zu erhalten; und
(c) Hinzufügen eines die Freisetzung regulierenden Agens oder eines pharmazeutisch annehmbaren Hilfsstoff zu dem Granula erhalten in Schritt (b) und Verpressen des resultierenden Gemisches zu einer Tablette.

2. Verfahren nach Anspruch 1, das ferner den Schritt des Hinzufügens eines Tablettierangshilfsstoffes, ausgewählt aus der Gruppe bestehend aus einem Zuckeralkohol, einem wasserlöslichen Polymer, einer öligen Base und eines Gemischs davon während der Zugabe der leichten wasserfreien Kieselsäure in Schritt (a) umfasst.

3. Das Verfahren nach Anspruch 1, wobei die verschmolzene feste Dispersion den aktiven Bestandteil und das pharmazeutisch annehmbare Adsorptionsmittel in einem Gewichtsverhältnis von 1 : 0,01 bis 1 : 3 umfasst.

4. Das Verfahren nach Anspruch 2, wobei die verschmolzene feste Dispersion den aktiven Bestandteil und den Tablettierungshilfsstoff in einem Gewichtsverhältnis von 1 : 0 bis 1 : 2 umfasst.

5. Das Verfahren nach Anspruch 2, wobei der Zuckeralkohol ausgewählt wird aus der Gruppe bestehend aus Xylitol, Sorbitol, Mannitol und einem Gemisch davon.

6. Das Verfahren nach Anspruch 2, wobei das wasserlösliche Polymer ausgewählt wird aus der Gruppe bestehend aus Hydroxypropylmethylcellulose, Hydroxypropylcellulose, Polyvinylpyrrolidon, Polyethylenglycol, Polyethylenoxid, Polyvinylalkohol und einem Gemisch davon.

7. Das Verfahren nach Anspruch 2, wobei die ölige Base ausgewählt wird aus der Gruppe bestehend aus Saccherose-Fettsäureester, Glycerylbehenat, Glycerylpalmitostearat, Glycerylmonooleat, Glycerylmonostearat und einem Gemisch davon,

8. Das Verfahren nach Anspruch 1, wobei die Tablette zur oralen Verabreichung eine Tablette zur kontrollierten Freisetzung ist, umfassend die verschmolzene feste Dispersion und ein die Freisetzung regulierendes Agens zur langsamen Freisetzung des aktiven Bestandteils;

9. Das Verfahren nach Anspruch 8, wobei die Tablette zur oralen Verabreichung ferner einen pharmazeutisch annehmbaren Träger umfasst.

10. Das Verfahren nach Anspruch 9, wobei die Tablette zur oralen Verabreichung die verschmolzene feste Dispersion, das die Freisetzung regulierende Agens und den pharmazeutisch annehmbaren Träger in einem Gewichtsverhältnis von 1 : 0,01 ∼ 3 : 0∼3 umfasst.

11. Das Verfahren nach Anspruch 1, wobei die Tablette zur oralen Verabreichung eine mehrschichtige Tablette ist, bestehend aus einer schnell freisetzenden Schicht umfassend die verschmolzene feste Dispersion und den pharmazeutisch annehmbaren Träger, und eine Schicht kontrollierten Freisetzung umfassend die verschmolzene feste Dispersion und ein die Freisetzung regulierendes Agens.

12. Das Verfahren nach Anspruch 8 oder 11, wobei das die Freisetzung regulierende Agens ausgewählt wird aus der Gruppe bestehend aus Polyethylenoxid mit einem Molekulargewicht von 10.000 bis 9.000.000, Hydroxypropylmethylcellulose mit einem Molekulargewicht von 1.000 bis 4.000.000, Hydroxypropylcellulose, Carboxyvinylpolymer, Polyvinylalkohol, Xanthan, Guarkernmehl, Johannisbrotkernmehl, Carboxymethylcellulose und ihre Derivate, Methylcellulose und ihre Derivate und Povidon-Polyvinylacetat Copolymer mit einem Molekulargewicht von 2.000 bis 2.000.000.

13. Das Verfahren nach Anspruch 11, wobei die die Schicht zur kontollierten Freisetzung ferner einen pharmazeutisch annehmbaren Träger umfasst.

14. Das Verfahren nach einem der Ansprüche 9, 11 und 13, wobei der pharmazeutisch annehmbare Träger ausgewählt wird aus der Gruppe bestehend aus einem quervernetzten Polyvinlypyrrolidon, einer quervernetzten Natriumcarboxymethylcellulose, Carboxymethylstärke, Calciummethacrylat-Divinylbenzen Copolymer, Polyvinylalkohol, Laktose, mikrokristalline Cellulose und Cellulosederivat, Stärke und ihre Derivate, Cyclodextrin und Dextrinderiväte, Quellstärke und ihre Derivate, kolloidales Siliziumdioxid, Magnesiumstearat, Glycerylmonostearat, Natriumstearylfumarat und Rizinusöl.

15. Das Verfahren nach Anspruch 11, wobei die schnell freisetzende Schicht ferner die verschmolzene feste Dispersion und den pharmazeutisch annehmbaren Träger in einem Gewichtsverhältnis von 1 : 0,05 bis 1 : 3 umfasst.

16. Das Verfahren nach Anspruch 13, wobei die regulierende Freisetzungsschicht die verschmolzene feste Dispersion, das die Freisetzung regulierende Agens und den pharmazeutisch annehmbaren Träger in einem Gewichtsverhältnis von 1 : 0,01 ∼ 3 : 0∼3 umfasst.

## Revendications

1. Procédé de préparation d'un comprimé destiné à l'administration par voie orale comprenant :
(a) le chauffage pour faire fondre un principe actif étant de l'ibuprofène, du dexibuprofène (S(+)-ibuprofène) ou un mélange de ceux-ci et l'ajout d'un adsorbant pharmaceutiquement acceptable étant un acide silicique anhydre léger présentant une surface spécifique allant de 20 à 400 m²/g à celle-ci pour obtenir une dispersion solide fusionnée homogène ;
(b) le refroidissement, le séchage et la pulvérisation de la dispersion solide fusionnée obtenue à l'étape (a) pour obtenir des granulés ; et
(c) l'ajout d'un agent de contrôle de la libération ou d'un excipient pharmaceutiquement acceptable aux granulés obtenus à l'étape (b) et la compression du mélange résultant sous la forme d'un comprimé.

2. Procédé selon la revendication 1, qui comprend en outre l'étape consistant à ajouter un auxiliaire de fabrication de comprimés choisi parmi le groupe comprenant un alcool glucidique, un polymère soluble dans l'eau, une base huileuse et un mélange de ceux-ci, au cours de l'ajout d'acide silicique anhydre léger lors de l'étape (a).

3. Procédé selon la revendication 1, dans lequel la dispersion solide fusionnée comprend le principe actif et l'adsorbant pharmaceutiquement acceptable suivant un rapport en poids allant de 1/0,01 à 1/3.

4. Procédé selon la revendication 2, dans lequel la dispersion solide fusionnée comprend le principe actif et l'auxiliaire de fabrication de comprimés suivant un rapport en poids allant de 1/0 à 1/2.

5. Procédé selon la revendication 2, dans lequel l'alcool glucidique est choisi parmi le groupe comprenant du xylitol, du sorbitol, du mannitol et un mélange de ceux-ci.

6. Procédé selon la revendication 2, dans lequel le polymère soluble dans l'eau est choisi parmi le groupe comprenant de l'hydroxypropylméthylcellulose, de l'hydroxypropylcellulose, de la polyvinylpyrrolidone, du polyéthylène glycol, de l'oxyde de polyéthylène, de l'alcool polyvinylique et un mélange de ceux-ci.

7. Procédé selon la revendication 2, dans lequel la base huileuse est choisie parmi le groupe comprenant de l'ester d'acide gras de sucrose, du béhénate de glycéryle, du palmitostéarate de glycéryle, du monooléate de glycéryle, du monostéarate de glycéryle et d'un mélange de ceux-ci.

8. Procédé selon la revendication 1, dans lequel le comprimé destiné à l'administration par voie orale est un comprimé à libération contrôlée comprenant la dispersion solide fusionnée et un agent de contrôle de la libération pour la libération lente du principe actif.

9. Procédé selon la revendication 8, dans lequel le comprimé destiné à l'administration par voie orale comprend en outre un excipient pharmaceutiquement acceptable.

10. Procédé selon la revendication 9, dans lequel le comprimé destiné à l'administration par voie orale comprend la dispersion solide fusionnée, l'agent de contrôle de la libération et l'excipient pharmaceutiquement acceptable suivant un rapport en poids allant de 1/0,01∼3/0∼3.

11. Procédé selon la revendication 1, dans lequel le comprimé destiné à l'administration par voie orale est un comprimé multicouche se composant d'une couche à libération rapide comprenant la dispersion solide fusionnée et l'excipient pharmaceutiquement acceptable, et d'une couche à libération contrôlée comprenant la dispersion solide fusionnée et un agent de contrôle de la libération.

12. Procédé selon les revendications 8 ou 11, dans lequel l'agent de contrôle de la libération est choisi parmi le groupe comprenant de l'oxyde de polyéthylène présentant un poids moléculaire allant de 10 000 à 9 000 000, de l'hydroxypropylméthylcellulose présentant un poids moléculaire allant de 1 000 à 4 000 000, de l'hydroxypropylcellulose, d'un polymère de carboxyvinyle, de l'alcool polyvinylique, de la gomme xanthique, de la gomme de guar, de la gomme de caroube, de la carboxyméthylcellulose et de son dérivé, de la méthylcellulose et de son dérivé, et d'un copolymère de povidone-polyvinylacétate présentant un poids moléculaire allant de 2 000 à 2 000 000.

13. Procédé selon la revendication 11, dans lequel la couche à libération contrôlée comprend en outre un excipient pharmaceutiquement acceptable.

14. Procédé selon l'une quelconque des revendications 9, 11 et 13, dans lequel l'excipient pharmaceutiquement acceptable est choisi parmi le groupe comprenant une polyvinylpyrrolidone réticulée, d'une carboxyméthylcellulose sodique réticulée, d'un amidon de carboxyméthyle, d'un copolymère de méthacrylate de calcium-divinylbenzène, d'alcool polyvinylique, de lactose, de cellulose microcristalline et d'un dérivé de cellulose, d'amidon et de son dérivé, de cyclodextrine et d'un dérivé de la dextrine, d'amidon prégélatinisé et de son dérivé, de silice colloïdale, de stéarate de magnésium, de monostéarate de glycéryle, de fumarate de stéaryle sodique et d'huile de ricin hydrogénée.

15. Procédé selon la revendication 11, dans lequel la couche à libération rapide comprend la dispersion solide fusionnée et l'excipient pharmaceutiquement acceptable suivant un rapport en poids allant de 1/0,05 à 1/3.

16. Procédé selon la revendication 13, dans lequel la couche à libération contrôlée comprend la dispersion solide fusionnée, l'agent de contrôle de la libération et l'excipient pharmaceutiquement acceptable suivant un rapport en poids allant de 1/0,01∼3/0∼3.
